# EUROPEAN PATENT APPLICATION

(11) **EP 0 937 470 A2**
(43) Date of publication of application: **25.08.1999**
(21) Application number: 99101762.5
(22) Date of filing: 11.02.1999
(51) Int. Cl.: A61M 3/00

(54) **Syringe for pharmaceutical products, particularly laxatives and the like**

(30) Priority: 18.02.1998 PA 98084471
(71) Applicant: World Pharmaceutical, S.A., Panama 5 (PA)
(72) Inventor: Saval Recuero, Ricaurte Enrique, Panama (PA)
(74) Representative: Modiano, Guido, Dr.-Ing.

(57) **Abstract**

The syringe for pharmaceutical products, particularly laxatives and the like, comprises a bottle (2) which contains the product and has at least one deformable portion and an injection cannula (5) which is provided with elements (4) for coupling at the mouth (3) of the bottle (2) and is provided with an upper membrane (7) for closing the dispensing port (8) which can be perforated at the time of use.

## Description

The present invention relates to a syringe for pharmaceutical products, particularly laxatives and the like.

Packages for pharmaceutical products are known which are constituted by a sealed bottle for containing the product and by a sterilized cannula which is screwed on at the mouth of the bottle to allow to inject the product.

The pharmaceutical products contained in these packages also include laxatives and the like, which are generally used to perform bowel cleansing of the patient before surgery.

These packages are not devoid of drawbacks, including the fact that they are subject to the risk of contamination when the cannula is screwed onto the bottle, that they require a preliminary assembly at the time of use, with a consequent waste of time, and that they entail additional costs for packaging the individual parts and for sterilizing the cannula.

The aim of the present invention is to eliminate the above-mentioned drawbacks of the prior art, by providing a syringe for pharmaceutical products, particularly laxatives and the like, which allows to eliminate the risk of external contamination of the laxative contained therein, in order to simplify the method of use and to prevent any tampering.

Within the scope of this aim, an object of the present invention is to obtain a syringe which is simple, easy to provide in practice, safe in use, effective in operation, and relatively of low cost.

This aim, these objects and others are achieved by the present syringe for pharmaceutical products, particularly laxatives and the like, which comprises a bottle for containing the product and an injection cannula, characterized in that said bottle has at least one deformable portion and in that said cannula is provided with means for coupling at the mouth of said bottle and is provided with an upper membrane for closing the dispensing port which can be perforated at the time of use.

Further characteristics and advantages of the present invention will become apparent from the detailed following description of a preferred but not exclusive embodiment of a syringe for pharmaceutical products, particularly laxatives and the like, according to the invention, illustrated only by way of non-limitative example in the accompanying drawings, wherein:

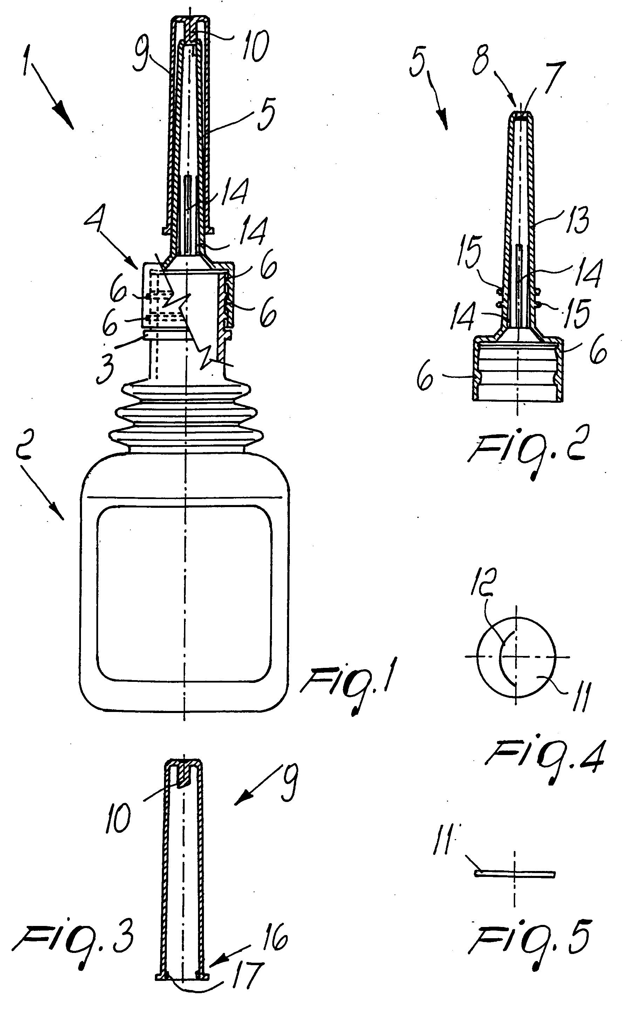

Figure 1 is a partially sectional front view of a syringe for pharmaceutical products, particularly laxatives and the like, according to the invention;
Figure 2 is a sectional view of the cannula of the syringe of Figure 1;
Figure 3 is a sectional view of the cannula covering cap of the syringe of Figure 1;
Figure 4 is a top view of the upper membrane for closing the syringe of Figure 1;
Figure 5 is a side view of the membrane of Figure 4.

With reference to the above figures, 1 generally designates a syringe for pharmaceutical products, particularly laxatives and the like, according to the invention.

The syringe 1 comprises a bottle 2 which contains the pharmaceutical product; an insertion cannula 5 is rigidly coupled to the mouth 3 of said bottle through a coupling means 4.

Advantageously, the bottle 2 is made of a deformable material which allows the user, by applying a slight pressure with his fingers, to convey the contained product into the cannula 5.

The coupling means 4 are constituted by a plurality of complementary annular profiled elements 6 which are provided on the internal surface of the cannula 5 and on the outer surface of the mouth 3.

The cannula 5 is provided, in an upward region, with a membrane 7 for blocking the dispensing port 8 which is adapted to prevent any external contaminants from entering the bottle 2.

A cannula covering cap 9 can be fitted over the cannula 5 and is provided, in an upward region, with a central internal pin 10 adapted to perforate at the time of use the membrane 7 that closes the cannula 5, by applying a slight downward pressure.

Conveniently, a membrane 11, adapted to prevent the reverse flow of the product, during the use of the syringe 1, is interposed between the mouth 3 of the bottle 2 and the cannula 5.

The membrane 11 has a substantially central slit 12 the width whereof allows the product to reach the dispensing port 8 of the cannula 5.

The cannula 5 is constituted by an elongated body 13, internally provided with a plurality of reinforcement ribs 14 and externally provided with two annular raised portions 15.

The mouth 16 of the cap 9 is centered between the raised portions 15 and in turn comprises a third annular internal raised portion 17 adapted to be arranged between the raised portions 15 of the cannula 5.

The invention thus conceived is susceptible of numerous modifications and variations, all of which are within the scope of the inventive concept.

All the details may further be replaced with other technically equivalent ones.

In practice, the materials employed, as well as the shapes and the dimensions, may be any according to requirements without thereby abandoning the scope of the protection of the appended claims.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A syringe for pharmaceutical products, particularly laxatives and the like, comprising a bottle (2) for containing the product and an injection cannula (5), characterized in that said bottle (2) has at least one deformable portion and in that said cannula (5) is provided with means (4) for coupling at the mouth (3) of said bottle (2) and is provided, in an upper region, with a membrane (7) for closing the dispensing port (8) which can be perforated at the time of use.

2. The syringe according to claim 1, characterized in that it is possible to fit a cannula covering cap (9) over said cannula (5), said cap (9) being provided, in an upward region, with a substantially central internal pin (10) adapted to perforate said closure membrane (7) before use.

3. The syringe according to one or more of the preceding claims, characterized in that a membrane (11) provided with slits (12) is interposed between the mouth (3) of said bottle (2) and said cannula (5) and is adapted to prevent the reverse flow of the product in operating conditions.

4. The syringe according to one or more of the preceding claims, characterized in that said cannula (5) has at least two annular raised portions (15), between which the mouth (16) of said cap (9) is centered.

5. The syringe according to one or more of the preceding claims, characterized in that said coupling means (4) are of the type with complementary annular profiles (6) provided on the internal surface of said cannula (5) and on the outer surface of the mouth (3) of said bottle (2).

6. The syringe according to one or more of the preceding claims, characterized in that said cannula (5) is provided, in a downward region, with a plurality of internal reinforcement ribs (14).
